(19) Europäisches Patentamt
European Patent Office
Office européen des brevets

(11) **EP 4 759 916 A1**

(12) **EUROPEAN PATENT APPLICATION**

(43) Date of publication:
**17.06.2026 Bulletin 2026/25**

(21) Application number: **25222226.0**

(22) Date of filing: **10.12.2025**

(51) International Patent Classification (IPC):
***C12M 1/00*** (2006.01)  ***C12M 3/00*** (2006.01)

(52) Cooperative Patent Classification (CPC):
**C12M 23/16; C12M 27/00; C12M 29/04;
C12M 35/02; C12M 35/04; C12M 41/00;
C12M 41/40; C12M 47/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **12.12.2024 LT 2024546**

(71) Applicant: **Valstybinis Moksliniu Tyrimu Institutas
Fiziniu
Ir Technologijos Mokslu Centras
02300 Vilnius (LT)**

(72) Inventors:
• **STANKEVIC, Voitech**
**07100 Vilnius (LT)**
• **STIRKE, Arunas**
**10322 Vilnius (LT)**
• **ABOUHAGGER, Adei Hussam Mohamed Gabr**
**02198 (LT)**

(74) Representative: **Draugeliene, Virgina Adolfina
Tarpine Ltd
A. P. Kavoliuko g. 24-152
04328 Vilnius (LT)**

(54) **DEVICE AND METHOD FOR PERMEABILISATION OF BIOLOGICAL CELLS IN CONDUCTIVE MEDIUM**

(57)    The invention concerns a microfluidic device designed for efficient extraction of valuable components from biological cells suspended in media with high electrical conductivity. The device consists of two channels (4, 5), a serpentine mixer (12), an electrical pulse treatment zone (6), and a passive variable-width chain-type mixer (13). A suspension, which is a medium containing cells (15) to be treated, is supplied through one channel (4), while a medium (16) is supplied through the other channel (5). The channels (4) and (5) are arranged one above the other and are interconnected in the electrical pulse treatment zone (6) by a porous substrate (3) that prevents the passage of cells. Two flat electrodes are arranged in this zone, one in the suspension channel (7a) and the other in the medium channel (7b). Due to the different flow velocities of the suspension and the medium, a layer of increased cell concentration (14) forms on the surface of the porous substrate (3), this layer having a higher electrical resistance than the medium. When a pulsed voltage is applied to the electrodes, the strongest electric field is generated at the surface of this layer (14), ensuring effective cell permeabilisation even in suspensions with high conductivity, such as marine algae media. After cell permeabilisation, intracellular components (17) are released as the suspension flows through the passive variable-width chain-type mixer (13). These components (17) pass through the porous substrate (3) and are discharged from the device together with the medium. This device design reduces suspension shunting effects and increases the efficiency of valuable material extraction from cells in conductive media.

Fig. 1

## Description

FIELD OF THE INVENTION

The invention relates to the fields of biotechnology and biophysics, specifically to technologies related to cell permeabilisation and extraction using electric fields, intended to modify biological objects or introduce substances into them. The invention is intended for use in laboratories and industrial biotechnology sectors related to marine algae, microorganisms, fungi and their mycelia, as well as plant and animal cells.

BACKGROUND OF THE INVENTION

Currently, one method for extracting valuable components from cells is permeabilisation. For this process, various chambers are used in which a suspension containing cells is placed between two electrodes that generate a strong pulsed electric field. However, known chambers of this type operate effectively only in media with low electrical conductivity.

For suspensions with high electrical conductivity, such chambers are unsuitable due to the shunting effect: the electric current flows predominantly through the medium, bypassing the cells, and thus the effectiveness of the electric field on the cells is significantly reduced. In media with high electrical conductivity, such as marine algae media, the electric current intensity between the electrodes is high, but the potential difference induced across cell membranes is too small. As a result, the cells are insufficiently affected, and the extraction of valuable substances from the cells becomes inefficient.

The present invention introduces an innovative device that addresses this problem by increasing the effectiveness of the electric field at the cells and enabling more efficient extraction of valuable substances.

Patent application No. US4910140 describes a method and apparatus for permeabilising cell walls using a pulsed electric field. The apparatus comprises a chamber with a pair of closely spaced electrodes, between which a sample - a suspension containing the cells to be permeabilised - is introduced. The electrodes are connected to a pulse generator that produces short electrical pulses. The volume of the processed sample depends solely on the electrode area and the distance between them. However, in an electrically conductive suspension medium, the shunting effect results in insufficient electric field impact on the cells. Furthermore, this chamber design does not permit the processing of large sample volumes.

Patent application No. US5690978 also relates to a device capable of continuously extracting valuable substances from cells using a pulsed electric field method. The device comprises at least two electrodes and an insulator. Each electrode is cylindrical and contains a flow chamber designed to establish electrical contact with the flowing suspension and to allow the suspension to pass through the processing device. The insulator between the electrodes is also cylindrical and includes a flow chamber located between the electrode flow chambers, allowing the pumped substance to flow from one electrode flow chamber to the other. A high-voltage pulse generator is connected to the electrodes. The electrodes and insulator flow chambers may have various cross-sectional and longitudinal geometries, including tubular, cylindrical, rectangular, elliptical, and non-uniform cross-sectional designs.

An improved continuous cell processing device is proposed in patent No. US9029108. However, the suspension processing chamber design in this invention has a drawback due to a highly non-uniform distribution of the electric field between the electrodes.

A device described in patent No. US11377652 is also known, consisting of two parallel electrodes between which the processed suspension flows. The device is intended for cell transfection using an electroporation apparatus that includes an electroporation chamber with an upper micro-mesh electrode, a lower micro-mesh electrode, and a channel formed in the electroporation chamber. These micro-mesh electrodes create a homogeneous electric field distribution throughout the processing chamber and allow the suspension to flow not only along the electrodes but also transversely through the electrode micro-mesh.

However, a drawback of the devices mentioned above is that after cell permeabilisation, the extraction products enter the same suspension flow and are not separated from unprocessed components. This issue may be addressed by using a membrane filter.

A microfluidic system described in patent No. US11724213 is known, which includes a chamber with a porous membrane filter designed to separate specific components from a suspension. It consists of two channels separated by a porous membrane with pores that allow only biological components of a certain size present in the suspension to pass through, while preventing the passage of larger cells. A suspension containing cells is supplied to the first channel, and only the suspension medium is supplied to the second channel. The membrane filter separates certain components from the fluid in the first channel and transfers them into the fluid in the second channel as both fluids flow through their respective channels. At the same time, components not permitted to pass through the membrane accumulate on the membrane surface of the first channel. With a hydrophobic membrane surface and fluid flow in both channels, a dynamic equilibrium layer with increased concentration forms on the membrane surface of the first channel. The concentration of components accumulated in the first channel and transferred into the second channel depends on the membrane characteristics and the flow rates of the fluids in both channels. However, such a design is suitable only for separating specific components.

It is known other microfluidic device used for epithelial cell growth and measuring their transepithelial impedance. The device features two microfluidic channels and electrodes positioned above and below a porous membrane that supports cell culture. This electrode arrangement ensures a uniform potential drop across the entire membrane (Odijk, van der Meer, Levner, Kim, van der Helm, Segerink, Frimat, Hamilton, Ingber, van den Berg, "Measuring direct current trans-epithelial electrical resistance in organ-on-a-chip microsystems," Lab on a Chip, vol. 15, issue 3, 7 February 2015, pp. 745-752; DOI: 10.1039/c4lc01219d).

Additionally, another microfluidic device and method are described in patent No. EP19701320, intended for investigating the electrical barrier properties of cells. This microfluidic device includes an electrically insulating porous support placed between electrodes. The support acts as a barrier to the electric field and prevents direct current from flowing from one electrode to the other through the suspension medium when a voltage is applied. The two electrodes may also be positioned on the inner surface of the device housing on the lower side of the flow channel. The device has inlet and outlet channels that form a path for the flow of the liquid medium through the flow channel. In this case, a voltage may be applied between the first and second electrodes to generate an electric field, and the electric current flows between the first and second electrodes and at least partially through the cell layer when a liquid medium is present in the flow channel and when a cell layer is grown on the porous support.

Another device is described in international patent application No. WO2015/181322, intended for measuring transepithelial electrical resistance in an *in vitro* cell barrier model. It consists of two channels separated by a porous membrane on which cells are cultured. Various types of cells forming a barrier may be grown on this membrane, for example, one or more cell types on the same side of the membrane or on both sides. The channels have inlet and outlet openings through which a suspension may flow, and two sets of electrodes are provided at the bottoms of the channels. The first set of electrodes generates an electric field, while the second set measures electrical potentials. An electrically conductive medium contacts these electrodes and creates an electric field in the cultured cell layer. The transepithelial electrical resistance of the cell layer is measured by applying alternating electrical voltages of different frequencies and measuring the voltage between the secondary electrodes.

The main drawback of such designs is that they are all intended for measuring certain parameters of cells grown on a membrane, rather than for exposing cells to an electric field.

The closest prior art to the present invention is a device described in patent application No. US2020115668, intended for cell processing, which uses a microfluidic chamber and has two flat micro-mesh-type electrodes with a semipermeable membrane arranged between them. This membrane is intended for the deposition of cells and other particles, and for their subsequent permeabilisation or electroporation. Such a design forms two channels: one intended for cell electroporation and the other for cooling the processed products.

However, as with the other devices listed above, the extraction products resulting from cell permeabilisation are not separated from the suspension flow.

BRIEF SUMMARY OF THE INVENTION

The objective of the present invention is to provide a cell permeabilisation device capable of efficiently modifying cells in high-conductivity suspensions, where, in a conventional chamber, the medium shunts the cells and the electric field applied to the cells is too weak to induce effective permeabilisation. This is particularly relevant when working with marine algae and other cells present in high-conductivity media, where a standard electrode configuration does not achieve sufficient cell permeabilisation efficiency.

The device proposed in the invention increases the efficiency of cell permeabilisation in media with high electrical conductivity by reducing electrical energy losses, as the majority of the electric field is directed onto the cells. This enables effective permeabilisation even in conductive suspensions that previously caused a shunting effect. The solution is advantageous for biotechnological processes requiring precise cell modification and extraction of valuable intracellular substances. The device design reduces the shunting effect and is therefore particularly suitable for processing marine algae and other biological objects frequently present in conductive environments. This technology not only saves energy and reduces the load on device components, but also increases overall process efficiency.

The invention concerns a microfluidic device designed for more efficient extraction of valuable components from biological cells present in media with high electrical conductivity. The device consists of two channels, a serpentine mixer, an electric-pulse processing zone, and a passive variable-width channel chain mixer. Through the upper channel, a suspension containing the cells to be processed is supplied, while through the lower channel, only the medium is supplied. In the electric-pulse processing zone, these channels are arranged one above the other and are connected by a porous support that prevents cells from passing through. Two planar electrodes are installed in this zone: one electrode is located in the suspension channel containing cells, and the other in the medium channel. Due to the different flow velocities of the suspension and the medium, a layer of increased cell concentration forms on the surface of the porous support, characterized by higher electrical resistance than the medium. When a pulsed voltage is applied to the electrodes, the strongest electric field is generated precisely at the surface of this increased cell concentration layer. This ensures effective cell permeabilisation even in high-conductivity suspensions, such as marine algae media. After cell permeabilisation, as the suspension flows through the passive variable-width channel chain mixer, intracellular components are released. These intracellular components pass through the porous support into the lower channel and are discharged from the device together with the medium. This device design reduces the medium shunting effect and increases the efficiency of extracting valuable substances from cells in a conductive medium.

BRIEF DESCRIPTION OF THE DRAWINGS

The drawings are provided solely as references to possible embodiments of the invention and do not limit its scope. None of the drawings or graphs presented should be considered limiting; they are merely illustrative examples of possible implementations of the invention.

Fig. 1 shows an exploded perspective view of the individual components of the device of the present invention.

Fig. 2 shows a perspective view of the upper and lower channels of the device and the positions of the electrodes.

Fig. 3 shows a schematic view of the device channels and the position of the porous support relative to them.

Fig. 4 shows a schematic view of the device channels and the locations of the cross-sections.

Fig. 5 shows a perspective sectional view of the device along line A-A in Fig. 4.

Fig. 6 shows a perspective sectional view of the device along line B-B in Fig. 4.

Fig. 7 shows a perspective sectional view of the device along line C-C in Fig. 4.

Fig. 8 shows a sectional view of the device along line D-D in Fig. 4 in the electric-pulse processing zone.

Fig. 9 shows a schematic view of the porous support, the layer of cells accumulated on it, and components extracted from the cells.

Fig. 10 shows an equivalent electrical circuit of the components during cell permeabilisation.

Fig. 11 shows a two-dimensional cross-section of the calculated electric field distribution between two electrodes.

Fig. 12 shows a one-dimensional cross-section of the calculated electric field distribution between two electrodes along line L shown in Fig. 11.

DETAILED DESCRIPTION OF THE EMBODIMENTS OF THE INVENTION

In this and the following embodiments, descriptions are provided with reference to the drawings. However, the invention is not limited to the embodiments presented; various modifications of the disclosed examples are possible.

To clearly and concisely illustrate embodiments of the invention, elements not directly related to the invention are not shown in the drawings. Identical or similar elements are denoted by the same reference numerals.

It should also be noted that the proportions of layers and elements in the drawings may be exaggerated or schematic, and some layers may be omitted for clarity. Furthermore, the dimensions of layers or elements shown may not correspond to their actual sizes, as they are schematic and intended to facilitate understanding of the device.

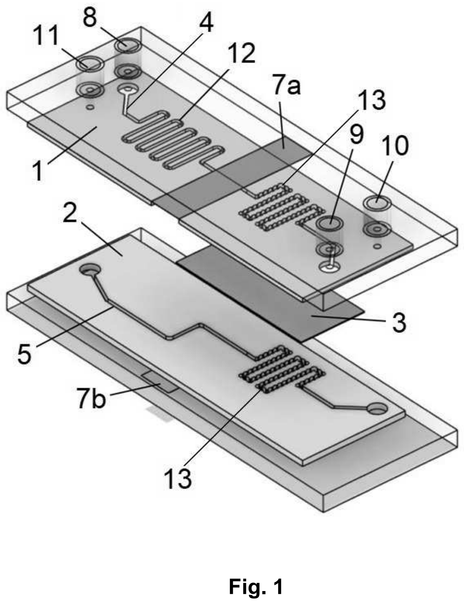

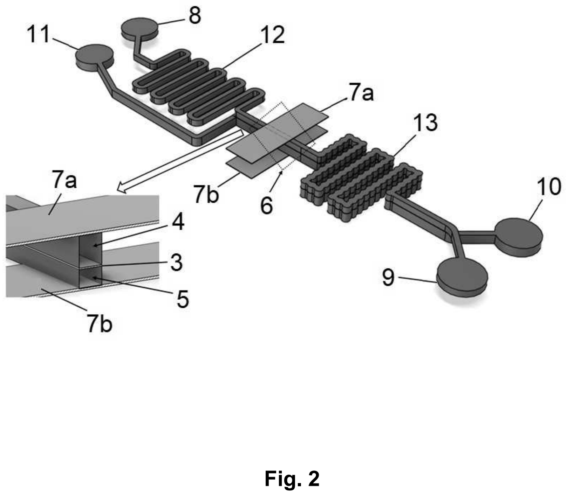

Fig. 1 shows an exploded perspective view of the device according to the present invention. In this embodiment, the device comprises two housing parts: an upper part 1 and a lower part 2, between which, at a specific location, a flat, thin, hydrophobic porous support 3, acting as a biological object filter, is arranged. The porous support 3, together with the upper housing part 1 and the lower housing part 2, forms two separate channels: an upper channel 4 and a lower channel 5. As shown in Fig. 2, the cross-sectional area of the lower channel 5 is smaller than that of the upper channel 4. In the electric-pulse processing zone 6, shown in Fig. 2, electrodes 7a and 7b are installed in the upper housing part 1 and the lower housing part 2.

A suspension consisting of a medium containing cells flows into the upper channel 4 through inlet 8 and exits through outlet 9. The medium is supplied to the lower channel 5 through inlet 10 and discharged through outlet 11. These fluids flow in opposite directions.

In this invention, the term "medium" refers to standard F/2 medium, consisting of approximately 95% seawater, with the remainder comprising various salts and vitamins. The term "cells" refers, without limitation, to marine algae, various microorganisms, fungi and their mycelia, as well as animal or plant cells.

Fig. 2 shows a perspective view of the configuration of the upper channel 4 and the lower channel 5, as well as the positions of electrodes 7a and 7b. From the medium inlet 10 up to and including the electric-pulse processing zone 6, the lower channel 5 replicates the configuration of the upper channel 4, but from this zone 6 to the medium outlet 11, the channels 4 and 5 are separated. Fig. 2 also shows, separately, a cross-sectional view of the upper and lower channels 4 and 5, and the porous support 3 at the location where the channels are connected.

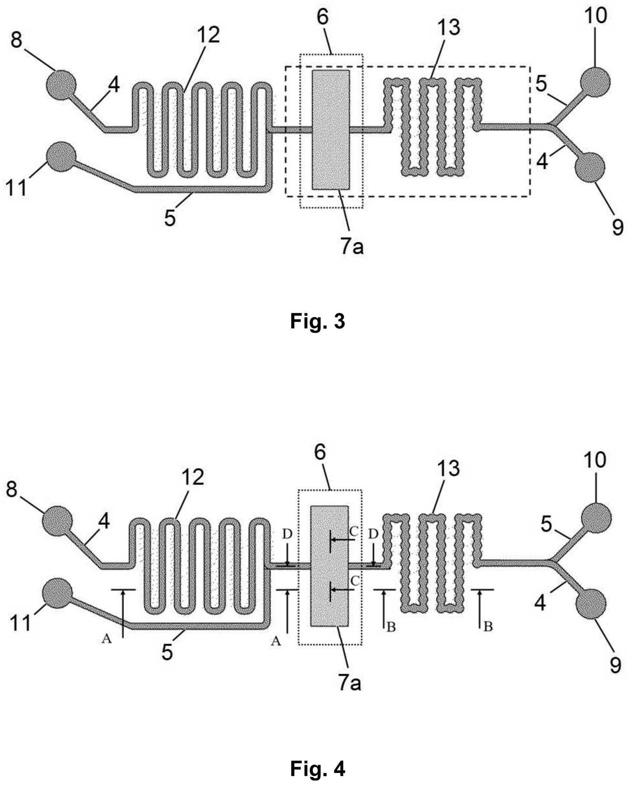

Fig. 3 shows a schematic top view of the upper and lower channels 4 and 5, and the position of the porous support 3 relative to them. The porous support 3 is inserted between the upper housing part 1 and the lower housing part 2 in the area defined by a dashed line, which includes the electric-pulse processing zone 6 and the chain mixer 13.

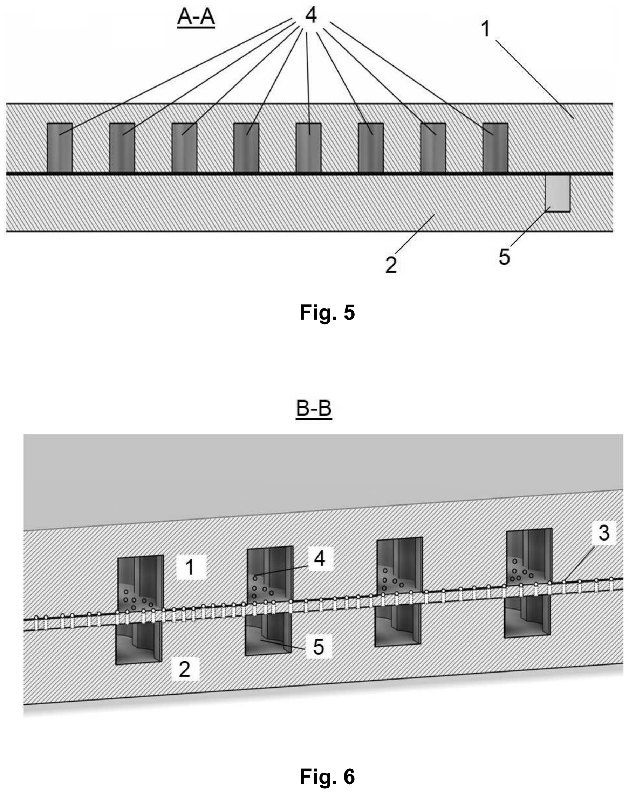

Fig. 4 shows a schematic top view of the upper and lower channels 4 and 5, as well as the locations of cross-sections A-A, B-B, C-C, and D-D, which are shown separately in Figs. 5-8.

Fig. 5 shows a perspective sectional view of the device along line A-A of Fig. 4. This section passes through the upper housing part 1 and the upper channel 4 in the serpentine mixer 12 region, as well as through the lower housing part 2 and the lower channel 5. At this point, the upper and lower channels 4 and 5 are separated.

Fig. 6 shows a perspective sectional view of the device along line B-B of Fig. 4. This section passes through the upper housing part 1 and the upper channel 4, and through the lower housing part 2 and the lower channel 5 in the variable-width channel chain mixer 13 region. At this point, the upper and lower channels 4 and 5 are connected via the porous support 3.

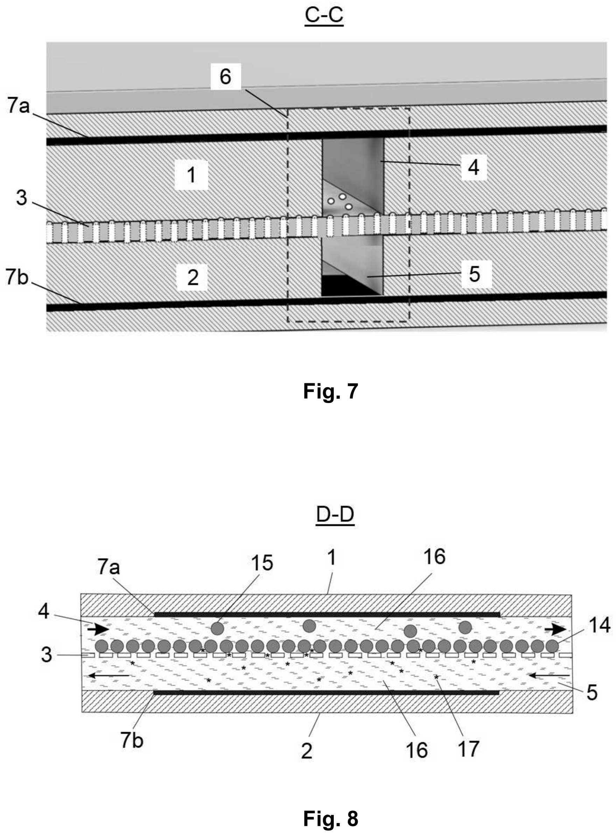

Fig. 7 shows a perspective sectional view of the device along line C-C of Fig. 4. This section passes through the upper housing part 1 and the upper channel 4, and through the lower housing part 2 and the lower channel 5 at the electric-pulse processing zone 6. At this point, the upper and lower channels 4 and 5 are connected via the porous support 3, and two electrodes are mounted in the upper portion of the upper channel 4 and the lower portion of the lower channel 5: the upper electrode 7a and the lower electrode 7b.

Fig. 8 shows a perspective sectional view of the device along line D-D of Fig. 4 at the electric-pulse processing zone 6. The figure shows the upper electrode 7a, the lower electrode 7b, the porous support 3, the layer of cells 14 accumulated on the surface of the porous support 3, individual cells 15 in the medium 16, and substances 17 extracted from the cells. The thickness of the porous support 3 may vary, with a preferred range of approximately 2 $\mu$m to 10 $\mu$m, and a porosity of 20% to 50%. The porous support 3 may be a commercially available membrane filter, such as a porous polymer film supplied by companies including, but not limited to, Advantec MFS, Inc. (Dublin, California) or Sterlitech Corporation (Kent, Washington). The porous support 3 serves two main functions: accumulation of processed cells 15 on its surface to form the cell layer 14, and separation of substances 17 extracted from the cells from the suspension, enabling their transfer to the lower channel 5.

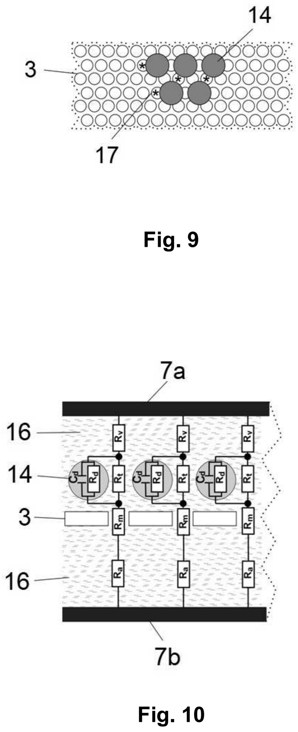

Fig. 9 shows a schematic view of the porous support 3 with the accumulated cell layer 14 and extracted intracellular substances 17. In this case, the pore diameter of the porous support 3 is smaller than that of the processed cells 14, so even under dynamic pressure between the upper and lower channels 4 and 5, the cells do not pass through the porous support 3. However, substances 17 extracted from the cells can freely enter the lower channel 5 of the device.

Fig. 10 shows a schematic sectional view of the device along line D-D of Fig. 4 at the electric-field processing zone, showing an equivalent electrical circuit of the cells accumulated on the porous support 3 and those in the medium. The equivalent circuit comprises the following components: upper and lower electrodes 7a and 7b, resistance Ra of the medium 16 in the lower channel 5, specific resistance Rs of the polymer porous support 3, resistance Rd and capacitive component Cd of the cell layer 14, and resistance Rv of the suspension in the upper channel 4. The figure also schematically shows micropores of the support 3, which have resistance Rm and through which the medium and substances extracted from the cells flow. Electric field distribution simulations were performed based on this equivalent circuit.

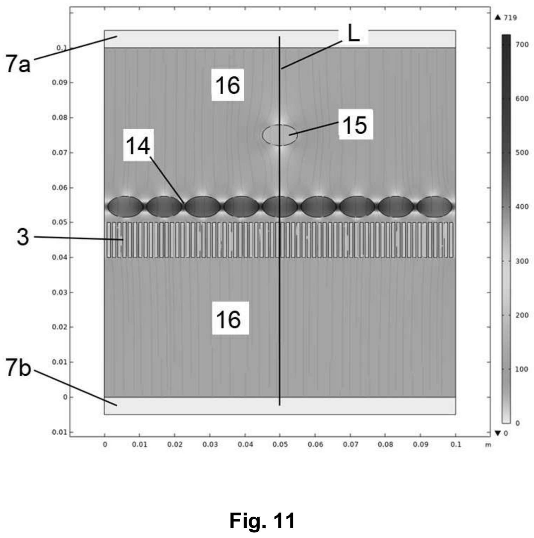

Fig. 11 shows a two-dimensional cross-section of the calculated electric field distribution between the two electrodes. The section intersects electrodes 7a and 7b, the medium 16, an individual cell 15, the cell layer 14, and the porous support 3. Color intensity corresponds to the electric field strength. As shown, the strongest electric field is present at the cell layer 14. The simulation results were used to optimize key parameters such as electrode configuration, electrode spacing, thickness of the accumulated cell layer, and medium conductivity.

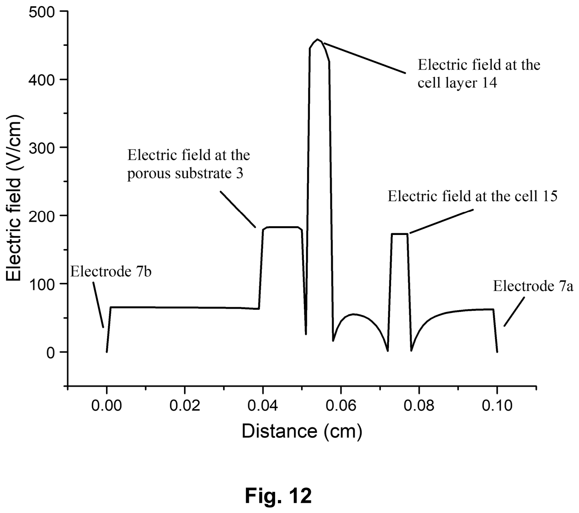

Fig. 12 shows a one-dimensional cross-section of the calculated electric field distribution between electrodes 7a and 7b along line L shown in Fig. 11. The section demonstrates that, for the selected device parameters and applied electrode voltage, the electric field at the cell layer 14 is three times stronger than at an individual cell 15 located in the conductive medium.

Method of Operation of the Device

As noted above, the invention concerns a microfluidic device designed for more efficient extraction of valuable components from biological cells in media with high electrical conductivity, particularly from microalgae. Among microalgae species, *Isochrysis galbana* is attracting increasing attention due to its high lipid content (20-30% of dry biomass), especially $\omega$-3 polyunsaturated fatty acids such as eicosapentaenoic acid (EPA, 20:5 $\omega$3) and docosahexaenoic acid (DHA, 22:6 $\omega$3). Other marine algae also yield substances such as fucoxanthin, astaxanthin, $\beta$-phycoerythrin, and fucoidans. When conventional devices are used, the high conductivity of the medium causes strong shunting, significantly reducing cell permeabilisation efficiency. This low efficiency results from the device design, in which a cell suspension with high medium conductivity is placed between two electrodes and subjected to an electric pulse. In such cases, the electric field generated at individual cells is weak due to the low resistance of the surrounding medium. The proposed device design addresses this by reducing the shunting effect through increased local cell concentration, thereby reducing the influence of the medium between the cells.

In the proposed device, a suspension consisting of an electrically conductive medium 16 and cells 15 to be processed, such as microalgae, is supplied to the inlet 8 of the upper channel 4. The suspension passes through a serpentine mixer 12, which occupies approximately one-third of the upper channel 4, where possible aggregates of the processed cells are disrupted due to multiple bends, and then enters the electric-pulse processing zone 6. At this point, the upper channel 4 is connected to the lower channel 5 through the porous support 3, through which pure medium 16 flows.

The upper and lower channels 4 and 5 have the same width; however, the height of the upper channel 4, through which the permeabilised suspension flows, is 20-40% greater than that of the lower channel. This design prevents clogging of the upper channel 4 by the suspension. The medium 16 supplied to the lower channel 5 through inlet 10 flows in the opposite direction to the permeabilised suspension in channel 4, and its velocity is 10-20% lower than the suspension velocity in the upper channel 4.

In general, the dynamic pressure in both channels is calculated according to the formula:

$$P_{dyn} = 0.5\, \rho\, v^2,$$

where $\rho$ is the fluid density and $v$ is its velocity. The main dynamic pressure difference between the channels can be described using the Bernoulli equation applied separately to each channel, taking the velocity difference into account:

$$\Delta P_{dyn} = 0.5 \cdot \rho_1 \cdot v_1{}^2 - 0.5 \cdot \rho_2 \cdot v_2{}^2,$$

where $\rho_1$ and $\rho_2$ are the fluid densities and $v_1$ and $v_2$ are their velocities.

Due to this velocity difference, higher pressure is created in the upper channel than in the lower channel, resulting in the accumulation of a layer of increased cell concentration 14 on the surface of the porous support. Furthermore, adjusting the relative flow velocities of the channels allows control of the thickness of the increased-concentration cell layer 14.

When a sufficiently high voltage - ranging from 1 kV to 2.5 kV at an electrode spacing of 1 mm - is applied to electrodes 7a and 7b, the strongest electric field in the electric-pulse processing zone 6 is generated at the cells 14 accumulated above the porous support 3. Thus, cell permeabilisation occurs specifically in this layer 14. At the same time, due to the hydrophobicity of the porous support 3 and the suspension flow, this cell layer 14 is not stationary, and permeabilised cells move into the passive variable-width channel chain mixer 13. In this part of the upper channel 4, periodic dynamic pressure is generated due to changes in channel width, promoting disruption of the cells 14 and release of intracellular components into the medium. Simultaneously, due to the pressure difference between the upper and lower channels 4 and 5, where the pressure is higher in the upper channel 4, these components readily pass through the porous support 3 and enter the lower channel 5. This suction process of extracted components occurs along the entire section where the upper and lower channels 4 and 5 are interconnected via the porous support 3. The products extracted from the cells are discharged together with the medium 16 through the medium outlet 11.

## Claims

1. A device for permeabilisation of biological cells in a conductive medium, comprising a chamber with two channels arranged one above the other, the channels being separated by a porous membrane, wherein a first channel is intended for a first medium containing cells to be permeabilised and a second channel is intended for a second medium, an electrical pulse generator, and two electrodes for exposing the first medium to electrical pulses, arranged above and below the two-channel chamber, **characterised in that** the device comprises:

   - an upper channel (4) for supplying a suspension to be permeabilised through an inlet (8) and for discharging the permeabilised suspension through an outlet (9);
   - a serpentine mixer (12) forming approximately one third of the upper channel (4), intended for homogenising the suspension of cells to be permeabilised flowing therein before entering an electrical pulse treatment zone (6);
   - an electrical pulse treatment zone (6) for the suspension of cells to be permeabilised, comprising the upper channel (4) and a lower channel (5) arranged one above the other and separated by a porous substrate (3), and an upper electrode (7a) and a lower electrode (7b) arranged above the upper channel (4) and below the lower channel (5), respectively;
   - a chain-type mixer (13) with a variable channel width, starting from the electrical pulse treatment zone (6) and ending at the outlet (9) of the permeabilised cell suspension, intended for generating periodic dynamic pressure and for releasing intracellular substances (17) from a permeabilised cell layer (14) in the upper channel (4) and transferring these substances into a medium (16) in the lower channel (5) through the porous substrate (3);
- a lower channel (5) for supplying the medium through an inlet (10) and for discharging the medium containing intracellular substances (17) extracted from the permeabilised suspension through an outlet (11).

2. The device according to claim 1, **characterized in that** the height of the upper channel (4) is greater than the height of the lower channel (5).

3. The device according to claim 2, **characterized in that** the height of the upper channel (4) is 20-40% greater than the height of the lower channel (5).

4. The device according to claim 1, **characterized in that** the porous substrate (3) is arranged only in the region between the upper channel (4) and the lower channel (5), this region comprising the electrical pulse treatment zone (6) and the chain-type mixer (13) zone.

5. The device according to claims 1 and 4, **characterized in that** the porous substrate (3) has a thickness of approximately 2 $\mu$m to 10 $\mu$m and a porosity of 20% to 50%.

6. The device according to claim 5, **characterised in that** the porous substrate (3) is a commercially available membrane filter, such as a porous polymer film.

7. A method for permeabilisation of biological cells in a conductive medium , comprising treating a first medium containing cells to be permeabilised with an electric field generated between two electrodes and withdrawing valuable intracellular substances separated through a membrane filter via a second medium, **characterised in that** the method comprises:

   - supplying a suspension of cells to be permeabilised through an inlet (8) into an upper channel (4) and supplying a medium through an inlet (10) into a lower channel (5), wherein the suspension and the medium flow in opposite directions and the flow velocity of the medium in the lower channel (5) is lower than the flow velocity of the suspension of cells to be permeabilised in the upper channel (4);
   - passing the suspension of cells to be permeabilised flowing in the upper channel (4) through a serpentine mixer (12), in which possible agglomerates of processed cells are disrupted due to multiple turns, thereby obtaining a homogenised cell suspension;
   - supplying the homogenised suspension of cells to be permeabilised into an electrical pulse treatment zone (6), in which the upper channel (4) and the lower channel (5) are arranged one above the other and separated by a porous substrate (3), applying a voltage to electrodes (7a, 7b), and performing cell permeabilisation;
- supplying the suspension containing permeabilised cells (14) into a chain-type mixer (13) zone, in which the upper channel (4) and the lower channel (5) are arranged one above the other and separated by the porous substrate (3), wherein periodic variation of the width of the upper channel (4) generates dynamic pressure that promotes disruption of the cell layer (14) and release of intracellular components (17) into the medium;
- transferring the released intracellular components (17) from the cell layer (14) through the porous substrate (3) into the lower channel (5);
- discharging the medium containing the released intracellular components (17) from the device through an outlet (11).

8. The method according to claim 7, **characterised in that** the flow velocity of the medium in the lower channel (5) is 10-20% lower than the flow velocity of the suspension of cells to be permeabilised in the upper channel (4), whereby, due to the difference in velocities, the pressure in the upper channel (4) is higher than in the lower channel (5), and the difference in dynamic pressures between the upper channel (4) and the lower channel (5) is calculated according to the Bernoulli equation:

$$\Delta P_{din} = 0.5{\cdot}\rho_1{\cdot}v_1{}^2 - 0.5{\cdot}\rho_2{\cdot}v_2{}^2,$$

where $\rho_1$ and $\rho_2$ are densities of the media, and $v_1$ and $v_2$ are their respective flow velocities.

9. The method according to claim 8, **characterised in that**, due to the increased pressure of the cell suspension in the upper channel (4), a layer of increased cell concentration (14) is formed on a surface of the porous substrate (3).

10. The method according to claims 8 and 9, **characterised in that**, by changing the flow velocities of the suspension and the medium flowing through the channels (4) and (5), the pressure of the suspension in the upper channel (4) and, correspondingly, the thickness of the layer of increased cell concentration (14) are adjusted.

11. The method according to claim 9, **characterised in that** the layer of increased cell concentration (14) has a higher specific electrical resistance than the suspension medium, enabling reduction of a cell shunting effect and creation of a stronger electric field, thereby ensuring more efficient cell permeabilisation.

12. The method according to claim 7, **characterised in that**, according to one embodiment of the invention, a voltage from 1 kV to 2.5 kV is applied to the electrodes (7a, 7b) in the electrical pulse treatment zone (6), while maintaining a distance of 1 mm between the electrodes.

**Fig. 1**

**Fig. 2**

**Fig. 3**

**Fig. 4**

11

**Fig. 5**

B-B

**Fig. 6**

C-C

**Fig. 7**

D-D

**Fig. 8**

**Fig. 9**

**Fig. 10**

**Fig. 11**

Fig. 12

Europäisches
Patentamt

European
Patent Office

Office européen
des brevets

## EUROPEAN SEARCH REPORT

**Application Number**

EP 25 22 2226

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2018/119296 A1 (CHARLES STARK DRAPER LABORATORY INC [US]) 28 June 2018 (2018-06-28) | 1-6 | INV.<br>C12M1/00<br>C12M3/00 |
| A | * paragraph [0006] *<br>* paragraph [0032] - paragraph [0084] * | 7-12 | |
| Y | WYATT SHIELDS IV ET AL: "Microfluidic cell sorting: a review of the advances in the separation of cells from debulking to rare cell isolation", LAB ON A CHIP, vol. 15, no. 5, 1 January 2015 (2015-01-01), pages 1230-1249, XP093393629, UK ISSN: 1473-0197, DOI: 10.1039/c4lc01246a * figure 10 * | 1-6 | |
| Y | NIU DONG ET AL: "Numerical investigation of multiscale lateral microstructures enhancing passive micromixing efficiency via secondary vortex flow", PHYSICS OF FLUIDS, vol. 34, no. 9, 1 September 2022 (2022-09-01), XP093393912, ISSN: 1070-6631, DOI: 10.1063/5.0105435 * introduction; figure 4 * | 1-6 | TECHNICAL FIELDS SEARCHED (IPC)<br><br>C12M<br>B01L |
| A | US 9 574 245 B2 (QVELLA CORP [CA]) 21 February 2017 (2017-02-21) * figure 3A * | 1-12 | |
| A | US 2022/126292 A1 (ESPINOSA HORACIO DANTE [US] ET AL) 28 April 2022 (2022-04-28) * figure 1 * | 1-12 | |

The present search report has been drawn up for all claims

| Place of search | Date of completion of the search | Examiner |
|---|---|---|
| The Hague | 1 May 2026 | Ledieu-Dherbécourt |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 22 2226

This annex lists the patent family members relating to the patent documents cited in  the above-mentioned European search report.
The members are as contained in the European Patent Office EDP file on
The European Patent Office is in no way liable for these particulars which are merely given for the purpose of information.

01-05-2026

| Patent document cited in search report | | Publication date | Patent family member(s) | | Publication date |
|---|---|---|---|---|---|
| WO 2018119296 | A1 | 28-06-2018 | US | 2018179485 A1 | 28-06-2018 |
| | | | US | 2020115668 A1 | 16-04-2020 |
| | | | WO | 2018119296 A1 | 28-06-2018 |
| US 9574245 | B2 | 21-02-2017 | CA | 2842720 A1 | 31-01-2013 |
| | | | EP | 2737047 A1 | 04-06-2014 |
| | | | ES | 2759824 T3 | 12-05-2020 |
| | | | JP | 6212488 B2 | 11-10-2017 |
| | | | JP | 6590865 B2 | 16-10-2019 |
| | | | JP | 2014521322 A | 28-08-2014 |
| | | | JP | 2018011590 A | 25-01-2018 |
| | | | US | 2014004501 A1 | 02-01-2014 |
| | | | US | 2017211128 A1 | 27-07-2017 |
| | | | US | 2020102596 A1 | 02-04-2020 |
| | | | WO | 2013013304 A1 | 31-01-2013 |
| US 2022126292 | A1 | 28-04-2022 | US | 2022126292 A1 | 28-04-2022 |
| | | | US | 20260008051 A1 | 08-01-2026 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4910140 A
- US 5690978 A
- US 9029108 B
- US 11377652 B
- US 11724213 B
- EP 19701320 A
- WO 2015181322 A
- US 2020115668 A

**Non-patent literature cited in the description**

- **ODIJK** ; **VAN DER MEER** ; **LEVNER, KIM** ; **VAN DER HELM** ; **SEGERINK** ; **FRIMAT, HAMILTON** ; **INGBER** ; **VAN DEN BERG**. Measuring direct current trans-epithelial electrical resistance in organ-on-a-chip microsystems. *Lab on a Chip*, 07 February 2015, vol. 15 (3), 745-752